(19) Europäisches Patentamt
European Patent Office
Office européen des brevets

(11) Numéro de publication : **0 299 890 B1**

(12) # FASCICULE DE BREVET EUROPEEN

(45) Date de publication du fascicule du brevet : **11.12.91 Bulletin 91/50**

(51) Int. Cl.$^5$ : **C07C 37/62**

(21) Numéro de dépôt : **88420242.5**

(22) Date de dépôt : **13.07.88**

(54) **Procédé de chloration de composés phénoliques.**

(30) Priorité : **17.07.87 FR 8710417**

(43) Date de publication de la demande : **18.01.89 Bulletin 89/03**

(45) Mention de la délivrance du brevet : **11.12.91 Bulletin 91/50**

(84) Etats contractants désignés : **AT BE CH DE ES FR GB GR IT LI LU NL SE**

(56) Documents cités :
**FR-A- 2 307 785**
**FR-A- 2 584 068**
**US-A- 4 160 114**
**US-A- 4 223 166**
**PATENT ABSTRACTS OF JAPAN, vol. 11, no. 43 (C-402)[2490], 7 février 1987, page 29 C 402; & JP-A-61 207 351**

(73) Titulaire : **RHONE-POULENC CHIMIE**
**25, quai Paul Doumer**
**F-92408 Courbevoie Cédex (FR)**

(72) Inventeur : **Desmurs, Jean-Roger**
**La Jonquière Route de Ternay**
**Communay§F-69360 St.-Symphorien d'Ozon (FR)**
Inventeur : **Besson, Bernard**
**15, rue de Moucherotte**
**F-38800 Pont De Claix (FR)**
Inventeur : **Jouve, Isabelle**
**30, rue Léon Fabre**
**F-69100 Villeurbanne (FR)**

(74) Mandataire : **Vignally, Noel et al**
**Rhône-Poulenc Interservices Service Brevets Chimie Centre de Recherches des Carrières**
**B.P. 62**
**F-69192 Saint-Fons Cédex (FR)**

**EP 0 299 890 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de chloration par le chlore gazeux de composés phénoliques substitués en positions ortho par rapport à la fonction hydroxyle.

Parmi les composés phénoliques importants, qui sont susceptibles d'être obtenus par chloration d'un composé phénolique substitué en positions ortho, se trouve le trichloro-2,4,6 phénol.

Le procédé habituel de préparation du trichloro-2,4,6 phénol consiste dans la chloration du dichloro-2,4 phénol.

Cependant il se forme une faible proportion de trichloro-2,4,5 phénol (de l'ordre de 0,003 à 0,010 % du poids de trichloro-2,4,6 phénol). Le trichloro-2,4,6 phénol, qui est un intermédiaire pour la synthèse d'autres composés, doit contenir le minimum de traces de cet isomère indésirable.

Une solution à ce problème consisterait donc à chlorer le dichloro-2,6 phénol, ce qui éviterait totalement la formation de trichloro-2,4,5 phénol. Le trichloro-2,3,6 phénol, susceptible de se former dans ce cas, à l'état de traces, est beaucoup moins gênant que le trichloro-2,4,5 phénol.

En fait, lorsque l'on chlore par le chlore gazeux le dichloro-2,6 phénol, on constate que l'on n'obtient pas un excellent rendement. Il se forme notamment une quantité importante de pentachloro-2,4,5,6,6 cyclohexène-2 one, ce qui rend le mélange réactionnel très instable et difficile à purifier.

La présente invention se propose de résoudre ce problème, ainsi que le problème plus général de la chloration en position para, avec un bon rendement, des composés phénoliques ayant des substituants en positions ortho par rapport à la fonction hydroxyle.

La demanderesse a proposé dans EP-A 0255452 un procédé de chloration par le chlore gazeux de composés phénoliques substitués en position ortho par rapport à la fonction hydroxyle, notamment le dichloro-2,6 phénol. Le procédé décrit dans EP-A 0255452 est caractérisé par le fait que l'on opère en présence d'une quantité efficace d'un acide fort ou d'un acide de Lewis.

Poursuivant ses recherches, la demanderesse a trouvé un nouveau procédé de chloration par le chlore gazeux de composés phénoliques de formule générale (I) :

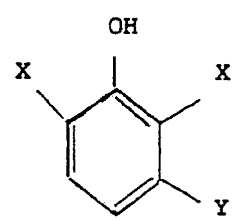

(I)

dans laquelle :

— les symboles X, identiques ou différents, représentent un atome de chlore, un atome de brome, un groupement méthyle ou éthyle, un groupement méthoxy ou éthoxy, un groupement acétoxy, un groupement $NO_2$, un groupement acylamino ayant 1 à 4 atomes de carbone,

— le symbole Y représente un atome d'hydrogène, un groupement méthyle ou éthyle, un groupement méthoxy ou éthoxy,

caractérisé en ce que l'on opère en présence d'une quantité efficace d'au moins un cation organique.

On peut mettre en oeuvre un cation organique ou un mélange de plusieurs cations organiques.

Par cation organique on entend dans le présent texte les ions onium dérivant notamment de l'azote, du phosphore, de l'arsenic, du soufre, du sélénium, de l'oxygène, du carbone ou de l'iode et coordiné à des restes hydrocarbonés. Les ions onium dérivant de l'azote, du phosphore ou de l'arsenic seront quadricoordinés, les ions onium dérivant du soufre, du sélénium, de l'oxygène, du carbone ou de S = 0 seront tricoordinés tandis que les ions onium dérivant de l'iode seront dicoordinés.

Les restes hydrocarbonés coordinés à ces différents éléments sont des radicaux alkyles, alcényles, aryles, cycloalkyles, aralkyles éventuellement substitués, 2 restes hydrocarbonés coordinés pouvant former ensemble un radical unique divalent.

La nature des anions liés à ces cations organiques n'a pas d'importance critique. Toutes les bases "dures" ou "intermédiaires" conviennent comme anion.

Par base "dure" ou "intermédiaire", on entend tout anion répondant à la définition classique donnée par R. PEARSON dans Journal of Chem. Ed. 45, pages 581-587 (1968), les termes "dure" et "intermédiaire" ayant respectivement la signification des termes de "hard" et "borderline" utilisés dans cette référence.

Parmi les ions onium pouvant être utilisés dans le présent procédé de chloration, conviennent particulièrement ceux répondant à l'une des formules générales suivantes :

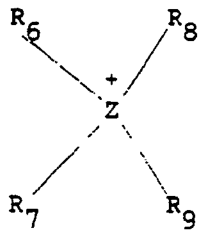

(II)

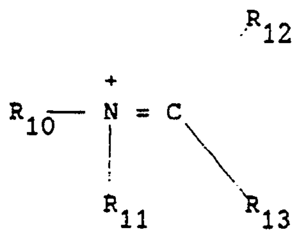

(III)

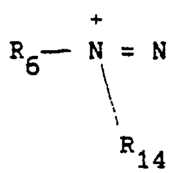

(III bis)

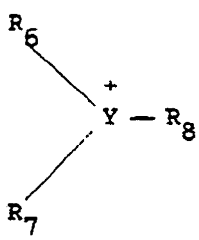

(IV)

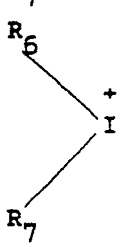

(V)

dans lesquelles :

— Z représente N, P ou As ;

— Y représente S, O, Se, S = 0 ou C ;

— $R_6$, $R_7$, $R_8$ et $R_9$, identiques ou différents représentent :

- un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
- un radical alcényle, linéaire ou ramifié, ayant 2 à 12 atomes de carbone ;
- un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène,
- deux desdits radicaux $R_6$ à $R_9$ pouvant former ensemble un radical alkylène, alcénylène ou alcadiè-

nylène, linéaire ou ramifié ayant de 3 à 6 atomes de carbone ;

— $R_{10}$, $R_{11}$, $R_{12}$, $R_{13}$ sont identiques ou différents et représentent :
  - un radical alkyle linéaire ou ramifié contenant de 1 à 4 atomes de carbone ;
  - les radicaux $R_{12}$ et $R_{13}$ pouvant former ensemble un radical alkylène contenant de 3 à 6 atomes de carbone ;
  - les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

— $R_{14}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_6$.

Parmi les bases "dures" ou "intermédiaires" pouvant constituer l'anion desdits sels d'onium, on peut citer les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle, ainsi que tous les autres anions répondant à la définition de base "dure" ou "intermédiaire" de PEARSON.

Pour des raisons de commodité de mise en oeuvre, lesdits anions pourront être choisis parmi $PO_4^{3-}$, $HPO_4^{2-}$, $H_2PO_4^-$, $CH_3SO_3^-$, $PH\text{-}SO_3^-$, $NO_3^-$, $SO_4^{2-}$, $PF_6^-$, $Cl^-$, $Br^-$, $I^-$,
Ph ayant la signification précédente. On recourra avantageusement aux anions $Cl^-$ et $Br^-$ et plus particulièrement à l'anion $Cl^-$.

A titre d'exemples de cations organiques répondant à la formule (II) on peut citer les cations :

— tétraméthylammonium,
— triéthylméthylammonium,
— tributylméthylammonium,
— triméthylpropylammonium,
— tétraéthylammonium,
— tétrabutylammonium,
— dodécyltriméthylammonium,
— méthyltrioctylammonium,
— heptyltributylammonium,
— tétrapropylammonium,
— tétrapentylammonium,
— tétrahexylammonium,
— tétraheptylammonium,
— tétraoctylammonium,
— tétradécylammonium,
— butyltripropylammonium,
— méthyltributylammonium,
— pentyltributylammonium,
— méthyldiétylpropylammonium,
— éthyldiméthylpropylammonium,
— tétradodécylammonium,
— tétraoctadécylammonium,
— hexadécyltriméthylammonium,
— benzyltriméthylammonium,
— benzyldiméthylpropylammonium,
— benzyldiméthyloctylammonium,
— benzyltributylammonium,
— benzyltriéthylammonium,
— phényltriméthylammonium,
— benzyldiméthyltétradécylammonium,
— benzyldiméthylhexadécylammonium,
— diméthyldiphénylammonium,
— méthyltriphénylammonium,
— butène-2-yltriéthylammonium,
— N,N-diméthyl-tétraméthylèneammonium,
— N,N-diéthyl-tétraméthylènammonium,
— tétraméthylphosphonium,
— tétrabutylphosphonium,

— éthyltriméthylphosphonium,
— triméthylpentylphosphonium,
— octyltriméthylphosphonium,
— dodécyltriméthylphosphonium,
— triméthylphénylphosphonium,
— diéthyldiméthylphosphonium,
— dicyclohexeyldiméthylphosphonium,
— diméthyldiphénylphosphonium,
— cyclohexyltriméthylphosphonium,
— triéthylméthylphosphonium,
— méthyltri(isopropyl)phosphonium,
— méthyltri(n-propyl)phosphonium,
— méthyltri(n-butyl)phosphonium,
— méthyltri(méthyl-2 propyl)phosphonium,
— méthyltricyclohexylphosphonium,
— méthyltriphénylphosphonium,
— méthyltribenzylphosphonium,
— méthyltri(méthyl-4 phényl) phosphonium,
— méthyltrixylylphosphonium,
— diéthylméthylphénylphosphonium,
— dibenzylméthylphénylphosphonium,
— éthyltriphénylphosphonium,
— tétraéthylphosphonium,
— éthyltri(n-propyl)phosphonium,
— triéthylpentylphosphonium,
— hexadécyltributylphosphonium,
— éthyltriphénylphosphonium,
— n-butyltri(n-propyl)phosphonium,
— butyltriphénylphosphonium,
— benzyltriphénylphosphonium,
— (β-phényléthyl)diméthylphénylphosphonium,
— tétraphénylphosphonium,
— triphényl(méthyl-4 phényl)phosphonium,
— tétrakis(hydroxyméthyl)phosphonium,
— tétraphénylarsonium.

Parmi les cations répondant aux formules (III) et (III bis) on peut citer les cations :
— N-méthylpyridinium,
— N-éthylpyridinium,
— N-hexadécylpyridinium,
— N-méthylpicolinium,
— triphényl-1,2,4 triazolium.

A titre d'exemples de cations organiques répondant à la formule (IV), on peut citer les cations :
— triméthylsulfonium,
— triéthylsulfonium,
— triphénylsulfonium,
— triméthylsulfoxonium,
— triphénylcarbénium,
— triéthyloxonium.

A titre d'exemples de cations organiques répondant à la formule (V), on peut citer les cations :
— diphényliodonium,
— diméthoxy-4,4′ diphényliodonium (ou les composés décrits dans JACS 1959, 81, 342),
— diphényliodonium 2-carboxylate.

Parmi les cations organiques qui peuvent être utilisés dans le cadre du présent procédé, on préfèrera le plus souvent les ions ammonium quaternaire, les ions phosphonium quaternaire, les ions sulfonium et les ions iodonium.

Le sel d'onium peut être introduit à l'état solide ou sous forme d'une solution dans l'un de ses solvants, le plus souvent l'eau.

Le procédé selon l'invention peut être conduit en l'absence de solvant : les réactifs sont alors à l'état fondu.

Cette variante de l'invention conduit généralement aux meilleurs résultats.

On peut également opérer dans un milieu liquide constitué notamment par un éther aliphatique, un hydrocarbure aliphatique, un hydrocarbure aliphatique chloré, un chlorobenzène ou un bromobenzène, un acide carboxylique.

A titre d'éther aliphatique, on peut citer notamment l'oxyde de dipropyle, l'oxyde de diisopropyle, l'oxyde de méthyle et de tertiobutyle.

A titre d'hydrocarbure aliphatique, on peut citer notamment l'hexane, l'heptane, l'octane, le nonane et le décane.

A titre d'hydrocarbure chloré, on peut citer les hydrocarbures perchlorés tels que notamment le tétrachlorure de carbone, le tétrachloroéthylène, l'hexachloroéthane, l'hexachloropropène et l'hexachlorobutadiène ; les hydrocarbures partiellement chlorés tels que le chlorure de méthylène, le dichloroéthane, le tétrachloroéthane, le trichloroéthylène, le chloro-1 butane, le dichloro-1,2 butane.

A titre de chlorobenzène, on peut citer en particulier le monochlorobenzène, le dichloro-1,2 benzène, le dichloro-1,3 benzène, le dichloro-1,4 benzène ou des mélanges de différents chlorobenzènes ; à titre de bromobenzène, on peut citer notamment le monobromobenzène ou des mélanges de monobromobenzène avec un ou plusieurs dibromobenzènes.

A titre d'acide carboxylique, on peut citer plus particulièrement l'acide acétique et l'acide propionique.

Lorsque le procédé de l'invention est mis en oeuvre en milieu solvant, la concentration du chlorophénol de formule (I) dans le solvant n'est pas critique. Elle dépendra notamment de la solubilité du chlorophénol dans le solvant utilisé.

La quantité de cation organique utilisée dans le procédé peut varier dans de très larges limites.

Habituellement elle représente en poids de sel d'onium par rapport au poids du composé phénolique de formule (I) de 0,005 % à 25 %.

Lorsque l'on opère à l'état fondu, on mettra en oeuvre préférentiellement de 0,015 % à 5 % en poids de sel d'onium par rapport au composé phénolique de formule (I), afin d'avoir une efficacité suffisante, tout en n'ayant pas une quantité excessive de sel d'onium.

Lorsque l'on opère en milieu solvant, on mettra en oeuvre préférentiellement de 5 à 25 % en poids de sel d'onium par rapport au composé phénolique de formule (I). Dans ce cas il est préférable d'exprimer la quantité de sel d'onium par rapport au mélange réactionnel. On utilisera par exemple de 0,01 % à 2 % en poids de sel d'onium par rapport au mélange réactionnel.

La quantité de chlore utilisée dans le procédé selon l'invention est essentiellement fonction du taux de transformation souhaité du composé phénolique (I).

En pratique, le plus souvent, le chlore est introduit par barbotage dans le milieu réactionnel. La pression dans l'appareillage est donc sensiblement égale ou légèrement supérieure à la pression atmosphérique.

Le chlore peut être utilisé seul ou être dilué par un gaz inerte, tel que l'azote par exemple. La présence d'un gaz inerte permet, si nécessaire, d'augmenter le débit gazeux sans augmenter corrélativement la quantité de chlore introduite en un temps donné.

Le chlore gazeux utilisé dans le présent procédé peut également être formé in situ, à partir d'acide chlorhydrique, par addition d'un composé oxydant, tel que par exemple le peroxyde d'hydrogène.

La température à laquelle est mis en oeuvre le procédé de l'invention est généralement inférieure ou égale à 180°C. La limite inférieure n'est pas critique. Elle est conditionnée par la nécessité d'avoir un mélange réactionnel liquide.

Lorsque l'on opère à l'état fondu, cette température inférieure variera donc selon le composé phénolique (I) soumis à la chloration. Ainsi lorsque l'on chlore le dichloro-2,6 phénol, il faudra une température d'au moins 65°C.

Lorsque l'on opère en milieu solvant, on pourra descendre la température jusqu'à 20°C par exemple.

De préférence cependant, la température sera comprise entre 40°C et 120°C, si l'on opère en milieu solvant.

Si l'on opère à l'état fondu, les températures préférées seront comprises entre 40 et 120°C, sauf bien entendu pour les composés phénoliques ayant un point de fusion supérieur à 40°C, pour lesquels la zone préférée de température sera comprise entre leur point de fusion et 120°C.

Parmi les composés phénoliques de formule (I) auxquels peut s'appliquer le procédé de l'invention, on peut citer plus particulièrement le dichloro-2,6 phénol, le diméthoxy-2,6 phénol, le chloro-2 méthoxy-6 phénol, le chloro-2 méthyl-6 phénol, le dichloro-2,6 méthyl-3 phénol, le dichloro-2,6 méthoxy-3 phénol, le bromo-2 méthoxy-6 phénol, le chloro-2 nitro-6 phénol, le chloro-2 acétamido-6 phénol.

On peut, si on le souhaite, chlorer des mélanges de ces composés phénoliques.

Le procédé de l'invention est tout particulièrement adapté à la chloration du dichloro-2,6 phénol en trichloro-2,4,6 phénol, car il permet d'obtenir ce dernier composé en limitant très fortement, généralement à moins

En fin de réaction l'essai est traité comme dans l'exemple 1.
On obtient les résultats suivants :

- taux de transformation (TT) du dichloro-2,6 phénol : 85,9 %
- rendement (RT) en trichloro-2,4,6 phénol par rapport au dichloro-2,6 phénol transformé : 90,0 %
- RT en tétrachloro-2,3,4,6 phénol : 1,2 %.

EXEMPLE 3

Dans l'appareillage décrit dans l'exemple 1, on charge :
— 42,66 g d'un mélange de chlorophénol comprenant :

| | | |
|---|---|---|
| . orthochlorophénol | : | 0,09 g (0,22 % en poids) |
| . dichloro-2,6 phénol | : | 10,08 g (23,63 % en poids) |
| . dichloro-2,4 phénol | : | 18,05 g (42,32 % en poids) |
| . trichloro-2,4,6 phénol | : | 11,21 g (26,28 % en poids) |
| . parachlorophénol | : | 0,57 g (1,35 % en poids) |
| . trichloro-2,4,5 phénol | : | 0,0822 % en poids |

(ce mélange est un brut de chloration industriel du phénol comprenant également des produits qui ne sont pas dosés)

— 0,42 g de chlorure de benzyl-diméthyl-hexadécylammonium.

Le mélange réactionnel est chauffé à 70°C sous agitation, puis on introduit 4,85 litres de chlore en 58 minutes 14 secondes.

En fin de réaction, le mélange réactionnel est traité et analysé comme dans l'exemple 1.

On obtient 50,26 g d'un mélange constitué de :

| | | |
|---|---|---|
| . dichloro-2,6 phénol | : | 0,23 g (0,46 % en poids) |
| . dichloro-2,4 phénol | : | 0,05 g (0,10 % en poids) |
| . trichloro-2,4,6 phénol | : | 46,91 g (93,33 % en poids) |
| . tétrachloro-2,3,4,6 phénol | : | 1,26 g (2,51 % en poids) |
| . tétrachloro-2,4,4,6 cyclohexadiénone | : | 0,5 g (0,99 % en poids) |
| . trichloro-2,4,5 phénol | : | 0,0019 % en poids |

On note la disparition presque totale du trichloro-2,4,5 phénol au cours de la chloration en présence du sel d'ammonium quaternaire.

ESSAI COMPARATIF A

Dans un appareillage semblable à celui de l'exemple 1, mais avec un réacteur de 500 $cm^3$, on charge :
— dichloro-2,6 phénol : 24,45 g (0,15 mole)
— éther isopropylique : 254 g

Le mélange réactionnel est chauffé à 70°C sous agitation et à cette température on introduit en 40 minutes 3,36 litres de chlore.

En fin de réaction l'essai est traité et analysé comme dans l'exemple 1.

On obtient les résultats suivants :

de 3 % en poids, la formation de produits secondaires indésirables tels que la pentachloro-2,4,5,6,6 cyclohexène-2 one.

Lorsque le procédé de l'invention est appliqué au dichloro-2,6 phénol, celui-ci peut être obtenu notamment par chloration à l'aide de chlore gazeux, de chloro-2 phénol, en présence d'un cation organique tel que défini précédemment.

On peut donc ainsi obtenir le trichloro-2,4,6 phénol par chloration du chloro-2 phénol par le chlore gazeux en présence d'un cation organique, qui catalysera tout d'abord la chloration du chloro-2 phénol en dichloro-2,6 phénol, puis la chloration de ce dernier en trichloro-2,4,6 phénol.

On peut également opérer selon la présente invention la chloration du dichloro-2,6 phénol préparé par chloration du phénol à l'aide de chlore gazeux, en présence d'un cation organique tel que défini précédemment.

Il s'avère que le procédé de l'invention s'applique également aux mélanges bruts de chloration du phénol, qui contiennent du dichloro-2,6 phénol ainsi que du dichloro-2,4 phénol, de l'orthochlorophénol et éventuellement de faibles quantités de parachlorophénol et de phénol.

Appliqué à de tels mélanges industriels, le procédé de l'invention permet d'obtenir avec un excellent rendement, pratiquement uniquement du trichloro-2,4,6 phénol. En outre on ne détecte pratiquement que très peu de trichloro-2,4,5 phénol, qui est un composé indésirable.

Les conditions décrites pour la chloration des composés phénoliques de formule (I) et plus particulièrement du dichloro-2,6 phénol, s'appliquent à la chloration du phénol et/ou du chloro-2 phénol ou des mélanges industriels bruts de chloration du phénol indiqués précédemment.

Les exemples qui suivent illustrent l'invention.

## EXEMPLE 1

Dans un réacteur en verre de 200 $cm^3$, équipé d'un agitateur, d'une tubulure plongeante permettant l'introduction de chlore gazeux, d'un thermomètre et surmonté d'un réfrigérant, on charge :

- dichloro-2,6 phénol : 32,6 g (0,2 mole)
- chlorure de tétrabutylammonium : 0,33 g (soit 1 % en poids par rapport au dichloro-2,6 phénol).

Le mélange réactionnel est chauffé sous agitation à 70°C et le chlore gazeux est alors introduit avec un débit de 5 l/h pendant 54 min, ce qui représente une quantité de chlore de 200 mmol.

En fin de réaction on purge l'appareil par un courant d'azote.

On analyse la masse réactionnelle par chromatographie en phase gazeuse (CPG) et par chromatographie liquide haute performance (CLHP).

On obtient les résultats suivants :

— taux de transformation (TT) du dichloro-2,6 phénol : 91,9 %
— rendement (RT) en trichloro-2,4,6 phénol par rapport au dichloro-2,6 phénol transformé : 97,1 %
— RT en tétrachloro-2,3,4,6 phénol : 0,6 %.
— RT en tétrachloro-2,4,4,6 cyclohexadiénone : 0,3 %
— trichloro-2,4,5 phénol : 0,0003 % en poids par rapport au mélange réactionnel.

## EXEMPLE 2

Dans un appareillage semblable à celui de l'exemple 1, mais avec un réacteur de 1000 $cm^3$, on charge :

- dichloro-2,6 phénol : 32,6 g (0,2 mole)
- tétrachloroéthylène : 612,5 g
- chlorure de tétrabutylammonium : 6,45 g (20 % en poids par rapport au dichloro-2,6 phénol).

Le mélange réactionnel est chauffé à 100°C et à cette température on introduit en 54 minutes 4,48 litres de chlore (0,2 mole).

— TT du dichloro-2,6 phénol : 0 %

ESSAI COMPARATIF B

Dans un appareillage semblable à celui de l'exemple 1, mais avec un réacteur de 500 $cm^3$, on charge :

- dichloro-2,6 phénol : 24,45 g (0,15 mole)
- tétrachlorure de carbone : 254 g

Le mélange réactionnel est chauffé à 75°C sous agitation et à cette température on introduit en 40 minutes 3,36 litres de chlore.

En fin de réaction l'essai est traité et analysé comme dans l'exemple 1.

On obtient les résultats suivants :

- TT du dichloro-2,6 phénol : 3 %
- RT en trichloro-2,4,6 phénol : 90 %

## Revendications

1. Procédé de chloration par le chlore gazeux de composés phénoliques de formule générale (I) :

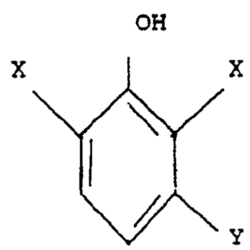

(I)

dans laquelle :

— les symboles X, identiques ou différents, représentent un atome de chlore, un atome de brome, un groupement méthyle ou éthyle, un groupement méthoxy ou éthoxy, un groupement acétoxy, un groupement $NO_2$, un groupement acylamino ayant 1 à 4 atomes de carbone,

— le symbole Y représente un atome d'hydrogène, un groupement méthyle ou éthyle, un groupement méthoxy ou éthoxy, caractérisé en ce que l'on opère en présence d'une quantité efficace d'au moins un cation organique.

2. Procédé selon la revendication 1, caractérisé en ce que l'on opère à l'état fondu.

3. Procédé selon la revendication 1, caractérisé en ce que l'on opère dans un solvant.

4. Procédé selon la revendication 3, caractérisé en ce que le solvant est constitué par un éther aliphatique, un hydrocarbure aliphatique, un hydrocarbure aliphatique chloré, un chlorobenzène ou un bromobenzène, un acide carboxylique.

5. Procédé selon l'une des revendications 1 à 4, caractérisé en ce que l'on opère en présence d'au moins un cation organique choisi les ions onium, répondant à l'une des formules générales suivantes :

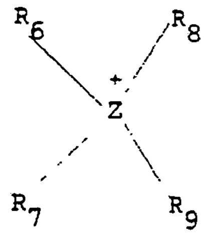

(II)

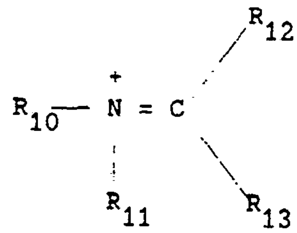

(III)

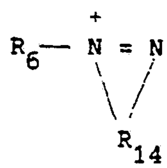

(III bis)

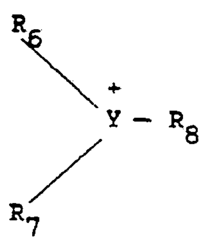

(IV)

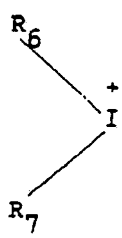

(V)

dans lesquelles :

— Z représente N, P ou As ;

— Y représente S, O, Se, S = O ou C ;

— $R_6$, $R_7$, $R_8$ et $R_9$, identiques ou différents représentent :

- un radical alkyle, linéaire ou ramifié, ayant 1 à 16 atomes de carbone et éventuellement substitué par un ou plusieurs groupements ou atomes phényle, hydroxyle, halogène, nitro, alkoxy ou alkoxycarbonyle, les groupements alkoxy ayant 1 à 4 atomes de carbone ;
- un radical alcényle, linéaire ou ramifié, ayant 2 à 12 atomes de carbone ;

- un radical aryle ayant 6 à 10 atomes de carbone, éventuellement substitué par un ou plusieurs groupements ou atomes alkyles ayant 1 à 4 atomes de carbone, alkoxy, alkoxycarbonyle, le radical alkoxy ayant 1 à 4 atomes de carbone, ou halogène,
- deux desdits radicaux $R_6$ à $R_9$ pouvant former ensemble un radical alkylène, alcénylène ou alcadiènylène, linéaire ou ramifié ayant de 3 à 6 atomes de carbone ;

— $R_{10}$, $R_{11}$, $R_{12}$, $R_{13}$ sont identiques ou différents et représentent :

- un radical alkyle linéaire ou ramifié contenant de 1 à 4 atomes de carbone ;
- les radicaux $R_{12}$ et $R_{13}$ pouvant former ensemble un radical alkylène contenant de 3 à 6 atomes de carbone ;
- les radicaux $R_{11}$ et $R_{12}$ ou $R_{11}$ et $R_{13}$ pouvant former ensemble un radical alkylène, alcénylène ou alcadiènylène, contenant 4 atomes de carbone et constituant avec l'atome d'azote un hétérocycle azoté ;

— $R_{14}$ représente un radical divalent formant avec les 2 atomes d'azote un cycle ayant 4 à 6 atomes pouvant comporter un ou plusieurs atomes d'azote, de soufre et/ou d'oxygène, ledit cycle pouvant être substitué par un ou plusieurs radicaux tels que $R_6$.

6. Procédé selon l'une des revendications 1 à 5, caractérisé en ce que l'anion lié au cation organique est choisi parmi les ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph représentant un radical phényle.

7. Procédé selon l'une des revendications 1 à 5, caractérisé en ce que l'anion lié au cation organique est choisi parmi les ions : $PF_6^-$, $PO_4^{3-}$, $HPO_4^{2-}$, $H_2PO_4^-$, $CH_3SO_3^-$, $Ph\text{-}SO_3^-$, $NO_3^-$, $SO_4^{2-}$, $Cl^-$, $Br^-$, $I^-$, Ph représentant un radical phényle.

8. Procédé selon l'une des revendications 1 à 7, caractérisé en ce que le cation organique est un ammonium quaternaire, un phosphonium quaternaire, un sulfonium ou un iodonium.

9. Procédé selon l'une des revendications 1 à 8, caractérisé en ce que l'on utilise de 0,005 % à 25 % en poids de sel d'onium par rapport au composé phénolique de formule (I).

10. Procédé selon la revendication 2, caractérisé en ce que l'on utilise de 0,015 % à 5 % en poids de sel d'onium par rapport au composé phénolique (I).

11. Procédé selon l'une des revendications 3 ou 4, caractérisé en ce que l'on utilise de 0,01 % à 2% en poids de sel d'onium par rapport au mélange réactionnel.

12. Procédé selon l'une des revendications 1 à 11, caractérisé en ce que le composé phénolique de formule (I) est choisi parmi le dichloro-2,6 phénol, le diméthoxy-2,6 phénol, le chloro-2 méthoxy-6 phénol, le chloro-2 méthyl-6 phénol, le dichloro-2,6 méthyl-3 phénol, le dichloro-2,6 méthoxy-3 phénol, le bromo-2 méthoxy-6 phénol, le chloro-2 nitro-6 phénol, le chloro-2 acétamido-6 phénol.

13. Procédé selon l'une des revendications 1 à 11, caractérisé en ce que le composé phénolique de formule (I) est le dichloro-2,6 phénol.

14. Procédé selon l'une des revendications 1 à 13, caractérisé en ce que l'on opère entre 40°C et 120°C ou entre le point de fusion du composé phénolique mis en oeuvre et 120°C.

15. Procédé selon la revendication 13, caractérisé en ce que le dichloro-2,6 phénol utilisé est obtenu par chloration à l'aide de chlore gazeux, de chloro-2 phénol, en présence d'un cation organique tel que défini dans les revendications précédentes.

16. Procédé selon la revendication 13, caractérisé en ce que le dichloro-2,6 phénol utilisé est obtenu par chloration à l'aide de chlore gazeux, de phénol, en présence d'un cation organique tel que défini dans les revendications précédentes.

17. Procédé selon l'une des revendications 1 à 16, caractérisé en ce qu'il est appliqué à des mélanges bruts de chloration du phénol, contenant du dichloro-2,6 phénol ainsi que du dichloro-2,4 phénol, de l'orthochlorophénol et éventuellement de faibles quantités de parachlorophénol et de phénol.

## Patentansprüche

1. Verfahren zum Chlorieren mittels Chlorgas von Phenolverbindungen der allgemeinen Formel (T) :

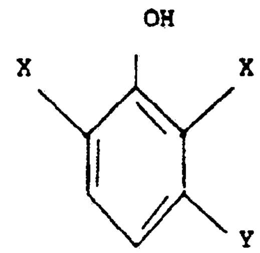

(I)

in der

— die Substituenten X gleich oder verschieden sind und jeweils für ein Chloratom, ein Bromatom, eine Methyl- oder Ethylgruppe, eine Methoxy- oder Ethoxygruppe, eine Acetoxygruppe, eine $NO_2$ -Gruppe, eine Acylaminogruppe mit 1 bis 4 Kohlenstoffatomen stehen,

— der Substituent Y für ein Wasserstoffatom, eine Methyl- oder Ethylgruppe oder für eine Methoxy- oder Ethoxygruppe steht, dadurch gekennzeichnet, daß man in Gegenwart einer wirksamen Menge mindestens eines organischen Kations arbeitet.

2. Verfahren nach Anspruch 1, dadurch gekennzeichnet, daß man in der Schmelze arbeitet.

3. Verfahren nach Anspruch 1, dadurch gekennzeichnet, daß man in einem Lösungsmittel arbeitet.

4. Verfahren nach Anspruch 3, dadurch gekennzeichnet, daß das Lösungsmittel ein aliphatischer Ether, ein aliphatischer Kohlenwasserstoff, ein aliphatischer Chlorkohlenwasserstoff, ein Chlorbenzol oder Brombenzol oder eine Carbonsöure ist.

5. Verfahren nach einem der Ansprüche 1 bis 4, dadurch gekennzeichnet, daß man in Gegenwart mindestens eines organischen Kations arbeitet, das aus den Oniumionen ausgewählt ist, die einer der folgenden allgemeinen Formeln entsprechen :

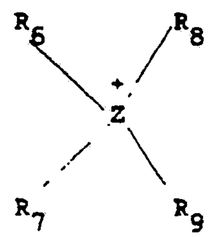

(II)

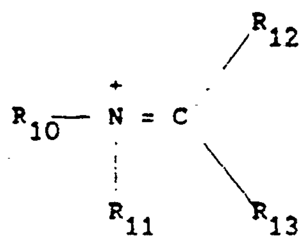

(III)

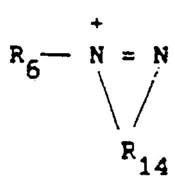

(III bis)

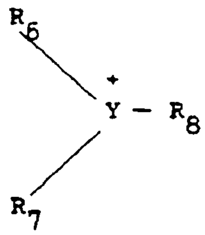

(IV)

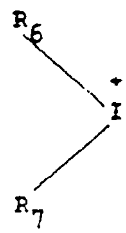

(V)

in denen

— Z für N, P oder As steht,

— Y für S, O, Se, S = 0 oder C steht,

— $R_6$, $R_7$, $R_8$ und $R_9$ gleich oder verschieden sind und bedeuten :

- einen linearen oder verzweigten Alkylrest mit 1 bis 16 Kohlenstoffatomen, gegebenenfalls substituiert durch eine/ein oder mehrere der folgenden Gruppen oder Atome Phenyl, Hydroxyl, Halogen, Nitro, Alkoxy oder Alkoxycarbonyl, wobei die Alkoxygruppen 1 bis 4 Kohlenstoffatome enthalten,
- einen linearen oder verzweigten Alkenylrest mit 2 bis 12 Kohlenstoffatomen,
- einen Arylrest mit 6 bis 10 Kohlenstoffatomen, gegebenenfalls substituiert durch eine/ein oder mehrere der folgenden Gruppen oder Atome Alkyl mit 1 bis 4 Kohlenstoffatomen, Alkoxy, Alkokycarbonyl, wobei der Alkoxyrest 1 bis 4 Kohlenstoffatome enthält, oder Halogen,
- zwei der Reste $R_6$ bis $R_9$ zusammen einen linearen oder verzweigten Alkylen-, Alkenylen- oder Alkadienylenrest mit 3 bis 6 Kohlenstoffatomen bilden können,

— $R_{10}$, $R_{11}$, $R_{12}$, $R_{13}$ gleich oder verschieden sind und bedeuten :

- einen linearen oder verzweigten Alkylrest mit 1 bis 4 Rohlenstoffatomen,
- die Reste $R_{12}$ und $R_{13}$ zusammen einen Alkylenrest mit 3 bis 6 Kohlenstoffatomen bilden können,
- die Reste $R_{11}$ und $R_{12}$ oder $R_{11}$ und $R_{13}$ zusammen einen Alkylen-, Alkenylen- oder Alcadienylenrest mit 4 Kohlenstoffatomen bilden können, der mit dem Stickstoffatom einen stickstoffhaltigen Heterozyklus bildet,

— $R_{14}$ einen zweiwertigen Rest, der mit den 2 Stickstoffatomen einen Zyklus bildet, der 4 bis 6 Atome aufweist, die ein oder mehrere Stickstoff-, Schwefel- und/oder Sauerstoffatome einschließen können, wobei dieser Zyklus durch einen oder mehrere Reste, wie $R_6$, substituiert sein kann.

6. Verfahren nach einem der Ansprüche 1 bis 5, dadurch gekennzeichnet, daß das an das organische Kation gebundene Anion ausgewählt wird aus : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, wobei Ph einen Phenylrest bedeutet.

7. Verfahren nach einem der Ansprüche 1 bis 5, dadurch gekennzeichnet, daß das an das organische Kation gebundene Anion aus folgenden I Jonen ausgewählt ist : $PF_6^-$, $PO_4^{3-}$, $HPO_4^{2-}$, $H_2PO_4^-$, $CH_3SO_3^-$, $Ph\text{-}SO_3^-$, $NO_3^-$, $SO_4^{2-}$, $Cl^-$, $Br^-$, $I^-$, wobei Ph einen Phenylrest bedeutet.

8. Verfahren nach einem der Ansprüche 1 bis 7, dadurch gekennzeichnet, daß das organische Kation ein quaternäres Ammonium, ein quaternäres Phosphonium, ein Sulfonium oder ein Iodonium ist.

9. Verfahren nach einem der Ansprüche 1 bis 8, dadurch gekennzeichnet, daß man 0,005 bis 25 Gew.-% Oniumsalz, bezogen auf die Phenolverbindung der Formel (I), einsetzt.

10. Verfahren nach Anspruch 2, dadurch gekennzeichnet, daß man 0,015 bis 5 Gew.-% Oniumsalz, bezogen auf die Phenolverbindung der Formel (I), einsetzt.

11. Verfahren nach einem der Ansprüche 3 oder 4, dadurch gekennzeichnet, daß man 0,01 bis 2 Gew.-% Oniumsalz, bezogen auf das Reaktionsgemisch, einsetzt.

12. Verfahren nach einem der Ansprüche 1 bis 11, dadurch gekennzeichnet, daß die Phenolverbindung der Formel (I) unter 2,6-Dichlorphenol, 2,6-Dimethoxyphenol, 2-Chlor-6-methoxyphenol, 2-Chlor-6-methylphe-

nol, 2,6-Dichlor-3-methylphenol, 2,6-Dichlor-3-methoxyphenol, 2-Brom-6-methoxyphenol, 2-Chlor-6-nitrophenol und 2-Chlor-6-acetamidophenol ausgewählt wird.

13. Verfahren nach einem der Ansprüche 1 bis 11, dadurch gekennzeichnet, daß die Phenolverbindung der Formel (I) 2,6-Dichlorphenol ist.

14. Verfahren nach einem der Ansprüche 1 bis 13, dadurch gekennzeichnet, daß man zwischen 40 und 120°C oder zwischen dem Schmelzpunkt der eingesetzten Phenolverbindung und 120°C arbeitet.

15. Verfahren nach Anspruch 13, dadurch gekennzeichnet, daß das eingesetzte 2,6-Dichlorphenol durch Chlorieren mittels Chlorgas von 2-Chlorphenol in Gegenwart eines organischen Kations, wie in den vorangegangenen Ansprüchen definiert, erhalten worden ist.

16. Verfahren nach Anspruch 13, dadurch gekennzeichnet, daß das eingesetzte 2,6-Dichlorphenol durch Chlorieren mittels Chlorgas, von Phenol in Gegenwart eines organischen Kations, wie in den vorangegangenen Ansprüchen definiert, erhalten worden ist.

17. Verfahren nach einem der Ansprüche 1 bis 16, dadurch gekennzeichnet, daß es auf rohe Gemische der Chlorierung von Phenol angewandt wird, die 2,6-Dichlorphenol sowie 2,4-Dichlorphenol, o-Chlorphenol und gegebenenfalls geringe Mengen an p-Chlorphenol und Phenol enthalten.

## Claims

1. Process, using gaseous chlorine, for the chlorination of phenolic compounds of general formula (I) :

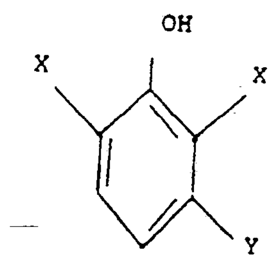 (I)

in which :

the symbols X, which can be identical or different, denote a chlorine atom, a bromine atom, a methyl or ethyl group, a methoxy or ethoxy group, an acetoxy group, an $NO_2$, group and an acylamino group having 1 to 4 carbon atoms, and

the symbol Y denotes a hydrogen atom, a methyl or ethyl group, a methoxy or ethoxy group, characterized in that it is carried out in the presence of an effective amount of at least one organic cation.

2. Process according to claim 1, characterized in that it is carried out in the molten state.

3. Process according to claim 1, characterized in that it is carried out in a solvent.

4. Process according to claim 3, characterized in that the solvent consists of an aliphatic ether, an aliphatic hydrocarbon, a chlorinated aliphatic hydrocarbon, a chlorobenzene or a bromobenzene, or a carboxylic acid.

5. Process according to any one of claims 1 to 4, characterized in that it is carried out in the presence of at least one organic cation chosen from amongst the onium ions, corresponding to one of the following general formulae :

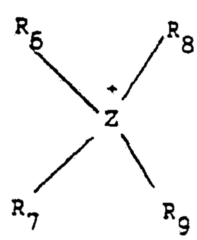 (II)

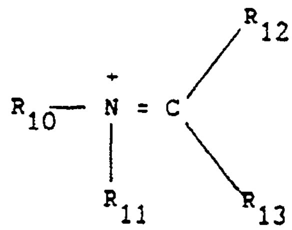

(III)

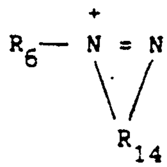

(III[1])

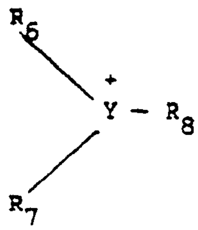

(IV)

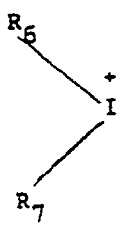

(V)

in which :

— Z denotes N, P or As ;

— Y denotes S, O, Se, S = 0 or C ;

— $R_6$, $R_7$, $R_8$ and $R_9$, which can be identical or different, denote :

a straight-chain or branched alkyl radical which has 1 to 16 carbon atoms and can be optionally substituted by one or more phenyl groups, hydroxyl groups, halogen atoms, nitro groups, alkoxy or alkoxycarbonyl groups, the alkoxy groups having 1 to 4 carbon atoms ;

a straight-chain or branched alkenyl radical having 2 to 12 carbon atoms ;

an aryl radical which has 6 to 10 carbon atoms and can be optionally substituted by one or more alkyl groups having 1 to 4 carbon atoms, alkoxy groups, alkoxycarbonyl groups, the alkoxy radical having 1 to 4 carbon atoms, or halogen atoms, two of the said radicals $R_6$ to $R_9$ being able to form together an alkylene, alkenylene or alkadienylene radical, which can be straight-chain or branched and has from 3 to 6 carbon atoms ;

— $R_{10}$, $R_{11}$, $R_{12}$, $R_{13}$ are identical or different and denote :

a straight-chain or branched alkyl radical containing 1 to 4 carbon atoms ; the radicals $R_{12}$ and $R_{13}$, being able to form together an alkylene radical containing from 3 to 6 carbon atoms ;

the radicals $R_{11}$ and $R_{12}$ or $R_{11}$ and $R_{13}$ being able to form together an alkylene, alkenylene or alkadienylene radical containing 4 carbon atoms and forming with the nitrogen atom a nitrogen-containing heterocycle ;

— $R_{14}$ denotes a divalent radical forming with the 2 nitrogen atoms a ring which has 4 to 6 atoms and can contain one or more nitrogen, sulphur and/or oxygen atoms, it being possible for the said ring to be substituted by one or more radicals such as $R_6$.

6. Process according to any one of claims 1 to 5, characterized in that the anion which is bound to the

organic cation is chosen from amongst the ions : $F^-$, $ClO_4^-$, $PF_6^-$, $BF_4^-$, $SnCl_6^-$, $SbCl_6^-$, $B(Ph)_4^-$, $PO_4^{3-}$, $HPO_4^{2-}$, $H_2PO_4^-$, $CH_3SO_3^-$, $Ph\text{-}SO_3^-$, $HSO_4^-$, $NO_3^-$, $SO_4^{2-}$, $Cl^-$, $Br^-$, $I^-$, $OH^-$, Ph denoting a phenyl radical.

7. Process according to any one of claims 1 to 5, characterized in that the anion which is bound to the organic cation is chosen from amongst the ions : $PF_6^-$, $PO_4^{3-}$, $HPO_4^{2-}$, $H_2PO_4^-$, $CH_3SO_3^-$, $Ph\text{-}SO_3^-$, $NO_3^-$, $SO_4^{2-}$, $Cl^-$, $Br^-$, $I^-$, Ph denoting a phenyl radical.

8. Process according to any one of claims 1 to 7, characterized in that the organic cation is a quaternary ammonium, a quaternary phosphonium, a sulphonium or an iodonium.

9. Process according to any one of claims 1 to 8, characterized in that 0.005% to 25% by weight of onium salt is used relative to the phenolic compound of formula (I).

10. Process according to claim 2, characterized in that 0. 015 % to 5 % by weight of onium salt is used relative to the phenolic compound (I).

11. Process according to either of claims 3 or 4, characterized in that 0.01% to 2% by weight of onium salt is used relative to the reaction mixture.

12. Process according to any one of claims 1 to 11, characterized in that the phenolic compound of formula (I) is chosen from amongst 2,6-dichlorophenol, 2,6-dimethoxyphenol, 2-chloro-6-methoxyhenol, 2-chloro-6-methylphenol, 2,6-dichloro-3-methylphenol, 2,6-chloro3-methoxyphenol, 2-bromo-6-methoxyphenol, 2-chloro-6-nitrophenol and 2-chloro-6-acetamidophenol.

13. Process according to any one of claims 1 to 11, characterized in that the phenolic compound of formula (I) is 2,6-dichlorophenol.

14. Process according to any one of claims 1 to 13, characterized in that it is carried out between 40°C and 120°C or between the melting point of the phenolic compound used and 120°C.

15. Process according to claim 13, characterized in that the 2,6-dichlorophenol used is obtained by chlorination of 2-chlorophenol, using gaseous chlorine, in the presence of an organic cation such as defined in the preceding claims.

16. Process according to claim 13, characterized in that the 2,6-dichlorophenol used is obtained by chlorination of phenol, using gaseous chlorine, in the presence of an organic cation such as defined in the preceding claims.

17. Process according to any one of claims 1 to 16, characterized in that it is applied to crude mixtures from the chlorination of phenol, containing 2,6-dichlorophenol as well as 2,4-dichlorophenol, orthochlorophenol and possibly small amounts of parachlorophenol and phenol.